# EUROPEAN PATENT APPLICATION

(11) **EP 4 360 836 A1**
(43) Date of publication of application: **01.05.2024**
(21) Application number: 21946598.6
(22) Date of filing: 14.07.2021
(51) Int. Cl.: B27N 3/12, B27N 3/18, B27N 3/20, B27J 7/00, B27K 9/00

(54) **JUNCAO FIBERBOARD AND PREPARATION METHOD THEREFOR**

(30) Priority: 24.06.2021 CN 202110705235
(71) Applicant: Zhongfu Strait (Pingtan) Development Co., Ltd., Fuzhou, Fujian 350400 (CN); Fujian Zhengyuan Juncao International Cooperation Co., Ltd., Fuzhou, Fujian 350001 (CN); Lin, Zhanxi, Fuzhou, Fujian 350001 (CN)
(72) Inventor: LIU, Pingshan, Fuzhou, Fujian 350400 (CN); LIN, Zhanxi, Fuzhou, Fujian 350001 (CN); LU, Maosheng, Fuzhou, Fujian 350400 (CN); LIN, Dongmei, Fuzhou, Fujian 350400 (CN); WANG, Zhiming, Fuzhou, Fujian 350400 (CN); LV, Xiangxi, Fuzhou, Fujian 350400 (CN); HE, Longqin, Fuzhou, Fujian 350400 (CN); XIONG, Fusheng, Fuzhou, Fujian 350400 (CN)
(74) Representative: Ipey
(86) International application number: PCT/CN2021/106323
(87) International publication number: WO 2022/267109

(57) **Abstract**

The present invention pertains to a method for preparing fungal mycelium fiberboards, which involves the following steps: cutting - cutting the 1-2 year-old fungal mycelium with a water content of 50-70% into lengths of 2-3 cm, to obtain the cut fungal mycelium; steaming - high-pressure steaming of the cut fungal mycelium to soften it, resulting in the steamed fungal mycelium; defibrillation and adhesive application - mechanically defiberizing the steamed fungal mycelium into fibers; adding adhesive to obtain adhesive-applied fungal mycelium fibers; drying - drying the adhesive-applied fungal mycelium fibers at 120-160°C, resulting in dried fungal mycelium fibers; spreading and pre-pressing - spreading, leveling, and pre-pressing to obtain fungal mycelium fiberboard blank; hot pressing - hot pressing the fungal mycelium fiberboard blank, resulting in the fungal mycelium fiberboard. Low-cost, fast-growing fungal mycelium is utilized as a raw material for the fungal mycelium fiberboard, which replaces wood chips. The preparation process is simple and easy to promote. The resulting fiberboard exhibits excellent performance in various indicators and is also environmentally friendly.

## Description

### BACKGROUND OF THE INVENTION

### 1. Field of the Invention

The present invention relates to the field of fiberboard technology, and particularly to a kind of fungal mycelium fiberboard and its preparation method.

### 2. Description of the Prior Art

China stands as the world's largest producer and exporter of engineered wood products. Annually, the demand for timber far surpasses its production. Engineered wood products are indispensable for the production and sustenance of our daily needs, playing a vital role in both the national economy and individual material lifestyles. In recent years, the depletion of forest resources and the increase in environmental awareness have necessitated the industry to conserve timber resources through efficient utilization, with the production and usage of engineered wood products serving as a primary means to achieve this.

Fiberboard primarily utilizes wood and its by-products from processing or non-wooden materials. The fibers are processed, combined with adhesive and other additives, and then hot-pressed to form a board with excellent cost-effectiveness and processability. The demand for fiberboard is on the rise, and its production is ranked first globally. The commonly used materials for medium-density fiberboard include pine, spruce, fast-growing poplar, and fast-growing eucalyptus. Its main constituents consist of wood fibers and resin adhesive.

In 2015, the government introduced a policy to comprehensively cease commercial logging in natural forests. As a result, the cost of timber gradually increased, and the supply-demand contradiction worsened. People began to seek alternatives to wood-based materials, and currently used non-wooden materials such as sugarcane face issues such as seasonal variations in raw material procurement and high procurement costs, which make large-scale production difficult. Moreover, environmental factors during the procurement season have a significant impact, resulting in unstable raw material quality. Due to the physical and chemical properties of sugarcane, it is impossible to produce high-quality medium-density fiberboard. Attempts have also been made to use herbaceous plants as a substitute for wood-based materials in board production, but they have all been unsuccessful in producing fiberboard that meets the quality and technical standards required by national and industry regulations. The effects of using grasses as a substitute for wood have been poor, likely due to the differences in stem structure and water content between woody and herbaceous plants.

Fast-growing carbon sequestration grass is a new variety developed through hybridization of certain grasses of the Poaceae family. With a short growth cycle, it can be harvested multiple times a year. Patent document CN 107032668 A provides a method for producing construction materials and wood substitutes using fast-growing carbon sequestration grass. The approach involves: (1) timely harvesting the fast-growing carbon sequestration grass; (2) pre-processing the grass according to the technical requirements for producing foam fiberboards, composite walls, lightweight bricks and tiles, flame-retardant insulation boards, wood substitutes, and paper substitutes; and (3) producing foam fiberboards, composite walls, lightweight bricks and tiles, flame-retardant insulation boards, wood substitutes, and paper substitutes. The products produced by this technology can replace lightweight materials such as composite materials and EPS foam plastics. They have a beautiful appearance, good sealing and buffering performance. However, the volume of the particleboard is relatively large, making it heavier than other types of boards. Therefore, it may not be suitable as a raw material for particleboard.

### Summary of the Invention

In response to this, the inventor provides a fiberboard made from natural fungal mycelium as raw material and its preparation method, aiming to replace wood-based raw materials and reduce production costs. Furthermore, the fiberboard produced using the preparation method of the present invention has enhanced toughness and is less prone to swelling in thickness due to water absorption.

To achieve the aforementioned objectives, the inventor has provided a method for preparing fungal mycelium fiberboards in a first aspect of the invention. The preparation method includes the following steps:
Cutting: Cut 1-2 year-old fungal mycelium with a moisture content of 50-70% into 2-3cm lengths to obtain cut fungal mycelium.
Steaming: high-pressure steaming the cut fungal mycelium to soften it, obtaining the steamed fungal mycelium;
Defibering and adhesive application: mechanically defiberizing the steamed fungal mycelium into fibers using a hot grinder. During the process of spraying fibers from the hot grinder, add adhesive to obtain adhesive-applied fungal mycelium fibers.
Drying: drying the adhesive-applied fungal mycelium fibers at 120-160°C, obtaining dried fungal mycelium fibers with a moisture content of 8.0-8.5%.

Spreading, leveling, and pre-pressing: spreading, leveling, and pre-pressing the dried fungal mycelium fibers to obtain fungal mycelium fiberboard blanks. The bulk density of the fungal mycelium fiberboard blanks should be controlled within 110-130kg/m³. As the bulk density of fungal mycelium fibers is nearly twice that of wood fibers, the spreading, leveling, and pre-pressing of fungal mycelium fiberboard blanks have different characteristics than wood fibers. The height of spreading, wind speed of leveling, wind pressure selection, and choice of pre-pressing net bags are all different from wood fibers.

Hot-pressing: hot-press the fungal mycelium fiberboard blank to obtain the fungal mycelium fiberboard.

Preferably, the fungal mycelium used is the Oasis 1 variety. Oasis 1 is a type of fungal mycelium belonging to the Poaceae family, resembling reeds and thin bamboo. It is a perennial herbaceous plant with thick and multi-jointed rhizomes, straight and tall stems that can branch, reaching a height of 3-10m with a stem diameter of 1-4cm, and large, green leaves measuring 2-5cm in width. Oasis 1 has fast growth, strong coverage, requires minimal management, and is free from pests and diseases. It is suitable for planting in various types of soil, including acidic sandy loam and mildly saline-alkali soils. It can be planted in drylands, wastelands, fields, mountain slopes, plains, terraces, riverbanks, lake shores, sandy areas, and other locations. After one planting, it can be harvested annually, with an expected yield of up to 20 years. The cellulose content of Oasis 1 rapidly increases to 30% after the heading stage, and the lignin content is 13-20%. The cellulose content continues to increase during the later growth stage. The 1-2 year-old fungal mycelium used in this invention has the optimal fiber content and fiber morphology, as experiments have shown that fungal mycelium with longer growth periods has higher lignin content.

Preferably, in the steaming step, the fungal mycelium is steamed at a temperature of 158-160°C, a steam pressure of 5.0-5.5 bar, and for a time period of 2-3 minutes. If the steam pressure is lower than 5.0 bar, it is not conducive to the slurry spraying of fungal mycelium fibers. If the steam pressure exceeds 5.5 bar or the steaming temperature is too high, the Oasis 1 fungal mycelium may undergo coking during the steaming process, resulting in brittle fiber loss and lack of toughness, which can affect the performance of the fiberboard. If the steaming temperature is too low, the fungal mycelium fibers may not soften sufficiently, which is not conducive to the later compression and stability of the finished fiberboard.

Preferably, in the defibering and adhesive application step, urea-formaldehyde resin is used as the adhesive, which has low levels of free formaldehyde and is an environmentally friendly adhesive.

Preferably, in the drying step, a flash-drying machine is used for drying.

The preferred hot-pressing step is carried out at a temperature of 153-163°C, a pressure of 170-180bar, and for a duration of 18-22s/mm. Controlling the hot-pressing temperature within this range, which is lower than the hot-pressing temperature of wood fiberboard, effectively prevents the charring of the fungal mycelium and ensures that the fungal mycelium fibers are in an optimal state of compression.

It is preferred that the fungal mycelium cuttings are steamed within two days after being cut. Storing the cuttings for longer than two days could result in fermentation or mold, which would cause a loss of raw materials.

In the second aspect of the invention, the inventor provides a fungal mycelium fiberboard, which is produced using the preparation method described in the first aspect of the invention.

Distinct from the existing technology, the technical solution provided herein offers a fungal mycelium fiberboard. The raw material for this fiberboard utilizes low-cost, fast-growing fungal mycelium with high cellulose content and desirable fiber morphology, as a substitute for wood-based materials. The preparation process is simple and easy to promote, resulting in a fiberboard with a dense layered structure, strong toughness, smooth and flat surface, minimal deformation, neat and delicate edges, uniform material color, and water absorption expansion rate and formaldehyde content that are superior to the technical indicators specified in GB/T 11718-2009. By using fast-growing fungal mycelium instead of wood as the raw material, the dependence on long cultivation cycle and limited logging utilization of trees in fiberboard manufacturing is significantly reduced, thus alleviating the contradiction between the demands of social-economic development and forest ecological environment balance.

### DETAILED DESCRIPTION OF THE INVENTION

In order to describe in detail the technical content, structural features, achieved objectives and effects of the instant application, the following detailed descriptions are given in conjunction with the drawings and specific embodiments. It should be understood that these embodiments are only used to illustrate the application and not to limit the scope of the instant application.

### Embodiment 1: An 18mm-thick fungal mycelium fiberboard and its preparation method

Cutting: Cut 1.5-year-old Oasis No.1 fungal mycelium with a water content of 50.2% into lengths of 2-3cm.
Steaming: Soften the cut fungal mycelium by high-pressure steaming; control the steaming temperature to be 158-160°C, steam pressure to be 5.0-5.5 bar, and the steaming time to be 2-3 min.
Defibering and adhesive application: Use a hot grinder to mechanically separate the fungal mycelium into fibers, and add adhesive during the spraying of fibers to obtain adhesive-applied fungal mycelium fibers. The amount of added adhesive is 10% of the mass of fungal mycelium after steaming, using urea-formaldehyde resin.
Drying: Use a flash-drying machine for air-drying of adhesive-applied fungal mycelium fibers at 120-160°C, and the moisture content of dried fungal mycelium fibers is 8.05%.

Spreading, leveling, and pre-pressing: spreading, leveling, and pre-pressing the dried adhesive-applied fungal mycelium fibers to obtain a fungal mycelium fiberboard blank. The compression rate of the dried fungal mycelium fibers after pre-pressing is 50-70%; the bulk density of the pre-pressed fungal mycelium is controlled at 110 kg/m³; the leveling roller height for the fungal mycelium fiberboard blank is 50-60% of that for wood fibers, and the height of the pre-pressed board blank is about 70% of that for wood fibers. The roller height of the fiberboard blank after leveling is about 50-60% of the height of the wooden fibers, and the height of the blank after pre-pressing is around 70% of the height of the wooden fibers.

Hot pressing: hot pressing the mycelium fiberboard billet at 153°C, a pressure of 170bar, and a time of 22s/mm to obtain the mycelium fiberboard.

Compared to using wood chips to make paper, the mycelium fiberboard preparation method provided by the present invention uses more flexible mycelium. In the hot grinder, the mycelium is actually defiberized, making the process simpler and increasing the utilization rate of mycelium, with almost no loss of cellulose. At the same time, the mycelium fibers in the fiberboard are coarser than traditional wood fibers, and less adhesive is used, making the resulting fiberboard more tough, heavier, and more environmentally friendly. However, the inventors found that the mycelium was prone to carbonization and cellulose loss during the preparation process, so the heating temperature during steaming, drying, and hot pressing must be strictly controlled to below 165°C, and preferably below 163°C.

The various properties of the mycelium fiberboard prepared in Embodiment 1 were tested according to GB/T11718-2009. The results are shown in Table 1:

**Table 1: Test results of 18mm thick mycelium fiberboard**

| No. | Check item | Unit | Standard value | Measured value |
|---|---|---|---|---|
| 1 | Moisture content | % | 3-13.0 | 6.2 |
| 2 | Modulus of rupture | MPa | µL : 24.0 | 24.0 |
| 3 | Modulus of elasticity | MPa | µL : 2300 | 3610 |
| 4 | Internal bond strength | MPa | µL : 0.45 | 1.17 |
| 5 | Water | % | µL : 12.0 | 6.9 |
| | absorption thickness swelling rate | | | |
| 6 | Surface bond strength | MPa | µL : 24.0 | 1.50 |
| 7 | Screw holding strength | N | - | Surface: 1690/ Edge:1380 |
| 8 | Formaldehyde emission | mg/ 100g | ≤8.0 | 5.0 |

### Embodiment 2: An 12mm-thick fungal mycelium fiberboard and its preparation method

In contrast to Embodiment 1, this embodiment involves the following steps:
Cutting: Cutting 2-year-old Oasis 1 fungal straw with a water content of 59.5% into lengths of 2-3cm;
Steaming: High-pressure steaming of the cut fungus to soften it; steaming temperature is maintained at 158-160°C, steam pressure is maintained at 5.0-5.5bar, and the steaming time is 2-3min;
Defibering and Adhesive application: Mechanically defibering the steamed fungus into fibers in a hot grinder, and spraying adhesive during the fiber spraying process to obtain adhesive-applied fungal straw fibers; the amount of adhesive added is 14.9% urea-formaldehyde resin based on the mass of the steamed fungus;
Drying: Using a flash-drying machine to dry the adhesive-applied fungal straw fibers at 120-160°C until the water content of the dried fungal straw fibers is 8.5%;
Spreading, Leveling, and Pre-Pressing: Spreading, leveling, and pre-pressing the dried fungal straw fibers to obtain the fungal mycelium fiberboard; the compression rate of the pre-pressing is 50-70%; and the bulk density of the fungus after pre-pressing is controlled at 130kg/m³;
Hot Pressing: Hot pressing the fungal mycelium fiberboard billet; the hot pressing temperature is 158°C, the hot pressing pressure is 180bar, and the hot pressing time is 18s/mm, resulting in the fungal mycelium fiberboard as described above.

Performance of the prepared fungal mycelium fiberboard in Embodiment 2 was tested according to GB/T11718-2009, and the results are presented in Table 2:

**Table 2. Test Results of 12mm-thick Agricultrual Waste Fiberboard**

| No. | Check item | Unit | Standard value | Measured value |
|---|---|---|---|---|
| 1 | Moisture content | % | 3-13.0 | 5.7 |
| 2 | Modulus of rupture | MPa | µL : 24.0 | 33.4 |
| 3 | Modulus of elasticity | MPa | µL : 2300 | 4010 |
| 4 | Internal bond strength | MPa | µL : 0.45 | 1.28 |
| 5 | Water absorption thickness swelling rate | % | µL : 12.0 | 7.8 |
| 6 | Surface bond strength | MPa | µL : 24.0 | 1.33 |
| 7 | Density | g/cm³ | / | 0.78 |
| 8 | Board Density Deviation | % | / | -5.1/+5.1 |
| 9 | Formaldehyde emission | mg/ 100g | ≤8.0 | 5.1 |

### Embodiment 3 - An 15mm-thick fungal mycelium fiberboard and its preparation method

In contrast to the previous two Embodiments, the following are the differences in Embodiment 3:
Cutting: 1-year-old Oasis No. 1 fungus with a water content of 54.5% was cut into lengths of 2-3cm;
Steaming: The cut fungus was steamed under high pressure to soften it. The steaming temperature was 158-160°C, the steam pressure was 5.0-5.5bar, and the steaming time was 2-3min;
Defibering and adhesive application: The fungus after steaming was mechanically defiberized in a hot grinder. During the process of spraying out the fibers in the hot grinder, adhesive was added to obtain adhesive-applied fungal fibers. The amount of adhesive added was 13.0% urea-formaldehyde resin by mass of the steamed fungus;
Drying: The adhesive-applied fungal fibers were dried in a flash-drying machine at 120-160°C, and the moisture content of the dried fungal fibers was 8.35%;
Spreading, leveling, and pre-pressing: The dried fungal fibers were laid, leveled, and pre-pressed to obtain fungal mycelium fiberboard blanks. The compression ratio of pre-pressing was 50-70%, and the volume weight of the fungal mycelium fiberboard blanks was controlled at 125kg/m³;
Hot pressing: The fungal mycelium fiberboard blanks were hot-pressed at a temperature of 163°C, a pressure of 175bar, and a hot pressing time of 20s/mm to obtain the fungal mycelium fiberboard.

### Embodiment 4: A 18mm-thick fungal mycelium fiberboard and its preparation method

The difference from Embodiment 1 lies in the steaming step, where the cut fungal mycelium is softened by high-pressure steaming. The steaming temperature is controlled at 150°C, the steam pressure at 4.8 bar, and the steaming time at 5 minutes. Due to the lower steaming temperature, the fungal mycelium fibers are not sufficiently softened, resulting in a smaller bulk density. Consequently, even though the subsequent process steps are the same, the resulting fungal mycelium fiberboard has a lower density, and the values of internal bond strength, water absorption thickness swelling rate, and surface bond strength are all lower than those of the fungal mycelium fiberboards obtained in Embodiments 1-3.

### Embodiment 5: An 18mm-thick fungal mycelium fiberboard and its preparation method

The difference from Embodiment 1 lies in the steaming step, where the cut fungal mycelium is softened by high-pressure steaming. The steaming temperature is controlled at 170°C, the steam pressure at 6 bar, and the steaming time at 3 minutes. Due to the excessively high steaming temperature and steam pressure, the fungal mycelium fibers suffer from carbonization loss, which affects the spraying process. Consequently, even though the subsequent process steps are the same, the resulting fungal mycelium fiberboard has lower values of modulus of rupture, internal bond strength, water absorption thickness swelling rate, and surface bond strength than those of the fungal mycelium fiberboards obtained in Embodiments 1-3.

### Embodiment 6: An 18mm-thick wood fiberboard and its preparation method

In this embodiment, fast-growing Eucalyptus saligna wood chips are used as raw materials. The cooking step is replaced by a hot grinding process, with the temperature controlled at 172-175°C, the cooking pressure at 7.5-8.0 bar, and the cooking time at 4-5 minutes. In the drying step, the moisture content of the wood fibers is controlled at 9.0-9.5%. During the mat forming, the height of the leveling roller for the wood fiberboard mat is 1.6-2 times the height of the fungal mycelium fiberboard mat, and the height of the pre-pressed wood fiberboard mat is 1.4-1.5 times that of the fungal mycelium fiberboard. In the hot pressing process, the hot pressing temperature of the wood fiberboard is 165-175°C, the maximum hot pressing pressure is 185-195 bar, and the hot pressing time is 15-18s/mm. The various test indicators of the wood fiberboard obtained by the above preparation method also meet the GB/T11718-2009 standard.

However, the inventor found through calculation that 15 tons of 5-year-old wood materials can produce 10m³ of fiberboard, and 15 tons of 2-year-old fungal mycelium materials can also produce 10m³ of fiberboard. From the perspective of the planting cycle, the output rate of fungal mycelium materials and the conversion rate of fiberboard are 2.5 times that of wood materials. From the perspective of logging utilization, fungal mycelium can be harvested multiple times per planting, with low investment costs in labor and fertilizers. From the perspective of ecological environmental sustainability, the substitution of fungal mycelium for wood materials in fiberboard production can effectively help to conserve forest trees and promote healthy and sustainable development of the ecological environment. Therefore, this technical solution of the invention has advanced technology and excellent economic and social benefits.

It should be noted that in this document, terms such as "first" and "second" are used solely to distinguish one entity or operation from another, and do not necessarily imply any actual relationship or sequence between these entities or operations. Additionally, the terms "including," "comprising," or any variations thereof are intended to encompass non-exclusive inclusion, such that processes, methods, items, or terminal devices comprising a series of elements include not only those elements explicitly listed, but also other elements that are inherently present in such processes, methods, items, or terminal devices. Unless otherwise specified, the use of the terms "greater than," "less than," "exceeding," etc. does not include the specified number, whereas the terms "above," "below," "within," etc. include the specified number.

Although the invention has been disclosed and illustrated with reference to particular embodiments, the principles involved are susceptible for use in numerous other embodiments that will be apparent to persons skilled in the art. This invention is, therefore, to be limited only as indicated by the scope of the appended claims.

## Claims

1. A method for preparing a fungal mycelium fiberboard, **characterized in that** the preparation method comprises a plurality of steps:
cutting: cutting 1-2 year-old fungal mycelium with a moisture content of 50-70% into 2-3 cm lengths, obtaining the cut fungal mycelium;
steaming: high-pressure steaming the cut fungal mycelium to soften it, obtaining the steamed fungal mycelium;
defibering and adhesive application: mechanically defiberizing the steamed fungal mycelium into fibers in a hot grinder; during the process of spraying fibers from the hot grinder, adding adhesive to obtain adhesive-applied fungal mycelium fibers;
drying: drying the adhesive-applied fungal mycelium fibers at 120-160°C, obtaining dried fungal mycelium fibers with a moisture content of 8.0-8.5%;
spreading, leveling, and pre-pressing: spreading, leveling, and pre-pressing the dried fungal mycelium fibers to obtain a fungal mycelium fiberboard blank, with the volume weight of the fungal mycelium fiberboard blank controlled at 110-130 kg/m3;
hot pressing: hot pressing the fungal mycelium fiberboard blank to obtain the fungal mycelium fiberboard.

2. The preparation method according to claim 1, **characterized in that** the fungal mycelium used is Oasis No.1 fungal mycelium.

3. The preparation method according to claim 1, **characterized in that** the steaming step has a steaming temperature of 158-160°C, a steam pressure of 5.0-5.5 bar, and a steaming time of 2-3 minutes.

4. The preparation method according to claim 1, **characterized in that** the defibering and adhesive application step uses urea-formaldehyde resin as the adhesive.

5. The preparation method according to claim 1, **characterized in that** the drying step uses a flash-drying machine for drying.

6. The preparation method according to claim 1, **characterized in that** the spreading, leveling, and pre-pressing step compresses the dried fungal mycelium fibers at a compression rate of 50-70%.

7. The preparation method according to claim 1, **characterized in that** the hot pressing step has a hot pressing temperature of 153-163°C, a hot pressing pressure of 170-180 bar, and a hot pressing time of 18-22 seconds/mm.

8. The preparation method according to claim 1, **characterized in that** the cut fungal mycelium is steamed within 2 days.

9. A fungal mycelium fiberboard, **characterized in that** the fungal mycelium fiberboard is produced using any one of the preparation methods according to claims 1-8.

10. The fungal mycelium fiberboard according to claim 9, **characterized in that** the density of the fungal mycelium fiberboard is 730-860 kg/m3.
